# FASCICULE DE BREVET EUROPEEN

(11) **EP 3 080 247 B1**
(45) Date de publication et mention de la délivrance du brevet: **28.02.2018**
(21) Numéro de dépôt: 14815303.4
(22) Date de dépôt: 12.12.2014
(51) Int. Cl.: C12N 5/078

(54) **CAPSULES CONTENANT DES CELLULES À POTENTIALITÉ HÉMATOPOÏÉTIQUE**
KAPSELN MIT ZELLEN MIT BLUTBILDUNGSPOTENZIAL
CAPSULES CONTAINING CELLS WITH BLOOD-FORMING POTENTIAL

(30) Priorité: 13.12.2013 FR 1362595
(43) Date de publication de la demande: 19.10.2016
(73) Titulaire: Etablissement Français du Sang, 93210 La Plaine Saint Denis (FR); Université Pierre et Marie Curie (Paris 6), 75005 Paris (FR); Ecole Supérieure de Physique et de Chimie Industrielles de la Ville de Paris, 75005 Paris (FR); Assistance Publique Hôpitaux de Paris, 75004 Paris (FR); Centre National de la Recherche Scientifique (C.N.R.S.), 75016 Paris (FR)
(72) Inventeur: GIARRATANA, Maria Catalina, F-93400 Saint Ouen (FR); DOUAY, Luc, F-75007 Paris (FR); DOMEJEAN, Hugo, F-75013 Paris (FR); BREMOND, Nicolas, F-75005 Paris (FR); BROUCHON, Julie Victoire, Cambridge, Massachussets 02139 (US); BAUDRY, Jean, F-75011 Paris (FR); BIBETTE, Jérôme, F-75005 Paris (FR)
(74) Mandataire: Lavoix
(86) Numéro de dépôt international: PCT/EP2014/077607
(87) Numéro de publication internationale: WO 2015/086832

(56) Documents cités:
- WO-A1-2008/149129
- WO-A1-2010/063937
- WO-A1-2011/101468
- MAZURIER C & DOUAY L: "Production de globules rouges dans un but transfusionnel ou l'itinéraire ex vivo d'une cellule souche", TRANSFUSION CLINIQUE ET BIOLOGIQUE, vol. 20, no. 2, 16 avril 2013 (2013-04-16) , pages 90-94, XP028542963, ISSN: 1246-7820, DOI: 10.1016/J.TRACLI.2013.02.020
- TIMMINS N E & NIELSEN L K: "Blood cell manufacture: current methods and future challenges", TRENDS IN BIOTECHNOLOGY, vol. 27, no. 7, 6 juin 2009 (2009-06-06), pages 415-422, XP055136498, ISSN: 0167-7799, DOI: 10.1016/j.tibtech.2009.03.008
- BREMOND N ET AL: "Formation of liquid-core capsules having a thin hydrogel membrane: liquid pearls", SOFT MATTER, vol. 6, no. 11, 3 mars 2010 (2010-03-03), pages 2484-2488, XP055072164, ISSN: 1744-683X, DOI: 10.1039/b923783f cité dans la demande
- YUAN Y ET AL: "Ex vivo amplification of human hematopoietic stem and progenitor cells in an alginate three-dimensional culture system", INTERNATIONAL JOURNAL OF LABORATORY HEMATOLOGY, vol. 33, no. 5, 14 avril 2011 (2011-04-14) , pages 516-525, XP055136502, ISSN: 1751-5521, DOI: 10.1111/j.1751-553X.2011.01324.x
- KIM C ET AL: "Generation of core-shell microcapsules with three-dimensional focusing device for efficient formation of cell spheroid", LAB ON A CHIP, vol. 11, no. 2, 21 janvier 2011 (2011-01-21), page 246, XP055050120, ISSN: 1473-0197, DOI: 10.1039/c0lc00036a

## Description

La présente invention concerne une capsule comprenant au moins une cellule à potentialité hématopoïétique, ladite capsule étant formée d'un coeur liquide et d'au moins une enveloppe gélifiée encapsulant totalement le coeur liquide à sa périphérie, l'utilisation d'une telle capsule pour la production *ex vivo* de cellules érythroïdes énucléées ainsi qu'une méthode *ex vivo* de production de cellules érythroïdes énucléées utilisant ladite capsule.

Il existe une demande toujours élevée de produits sanguins labiles, notamment à des fins de transfusion, et cette demande n'est pas satisfaite de manière satisfaisante par les approvisionnements actuels de sang humain naturel. En conséquence, de nombreux substituts au sang naturel ont été étudiés.

Cependant, les hémoglobines recombinantes ou stabilisées ont montré des performances décevantes, les indications de transporteurs artificiels d'oxygène sont limitées et le développement des globules rouges «universels» rendus compatibles avec le système ABO et/ou l'antigène RhD par traitement enzymatique ou masquage antigénique est lent.

Il existe donc un besoin d'alternatives à ces méthodes. À cet égard, les tentatives pour produire des cellules érythroïdes, tels que les globules rouges, à partir de cellules souches *in vitro,* sont particulièrement encouragées.

Toutefois, il s'agit d'un défi considérable que de reproduire *in vitro* ce qu'il faut à la nature plusieurs mois pour construire *in vivo.* Au cours de son développement chez les humains, l'érythropoïèse évolue à partir du mésoderme en deux vagues. L'érythropoïèse primitive commence dès la troisième semaine de gestation dans le sac vitellin (tissu extra-embryonnaire) et donne lieu à des érythrocytes primitifs nucléés, mégaloblastiques, qui synthétisent l'hémoglobine embryonnaire du type Gower I (ζ2ε2) et Gower II (α2ε2). L'érythropoïèse définitive commence au cours de la cinquième semaine de gestation dans la région de l'aorte-gonades mésonéphros (AGA), avant de migrer vers le foie foetal et la moelle osseuse. Les cellules érythroïdes matures produisent peu à peu, conduisant à la production de globules rouges énucléés (RBC), normocytaires et contenant de l'hémoglobine foetale (α2γ2) puis adulte (α2β2).

À ce jour, plusieurs tentatives de produire des globules rouges à partir de cellules souches embryonnaires humaines ont été rapportées, tels que décrites par Ma et al., (2008), Proc. Natl. Acad. Sci. USA, 105:13087-13092. Cependant, ces expériences reposent généralement sur une étape de co-culture en présence de cellules stromales, ce qui rend l'intensification des processus difficile.

On peut citer par exemple la demande de brevet WO2005118780 qui décrit une méthode *in vitro* pour la production massive et sélective d'érythrocytes énucléés. Selon cette méthode, les cellules souches hématopoïétiques sont cultivées dans un milieu de culture qui comprend au moins un facteur de croissance hématopoïétique puis la culture des cellules ainsi obtenue est mise en contact avec des cellules support.

La demande de brevet WO2011101468 décrit quant à elle un milieu de culture cellulaire, sans l'exigence d'une co-culture sur un stroma cellulaire, pour la croissance et/ou la différenciation des cellules de la lignée hématopoïétique qui comprend de l'insuline, de la transferrine et du plasma ou du sérum.

La méthode pour la production à l'échelle industrielle de choix est aujourd'hui le réacteur à agitation utilisé par exemple pour la production de vaccins ou d'anticorps monoclonaux, avec croissance des cellules en solution ou sur microparticules pour les cellules adhérentes.

Ce mode de production pose néanmoins des problèmes dès lors que les cultures sont réalisées à des densités de cellules importantes, ce qui est nécessaire pour une production industrielle. En effet, plus la densité de cellules est importante, plus il faut augmenter les flux et agiter en conséquence. Cependant, contrairement aux levures et aux bactéries, les cellules de mammifères, notamment les cellules souches, sont fragiles et résistent moins bien à ces traitements mécaniques, conduisant à des rendements beaucoup plus faibles qu'attendu.

Des billes remplies d'alginate sont utilisées en culture cellulaire, comme support pour des cellules adhérentes où les cellules sont donc situées à l'extérieur des billes.

Par la suite, l'idée d'encapsuler des cellules à l'intérieur de billes pleines d'alginate a été abordée. Un premier problème de toxicité s'est alors présenté dans le processus d'encapsulation et de nombreux développements sur le matériau et le processus de gélification ont été proposés pour améliorer la viabilité des cellules ainsi prises dans la matrice d'alginate (*A method for the large-scale cultivation of animal cells wherein animal cells are embedded in a collagen gel which is covered by a protective coating., The protective coating supports and protects the collagen matrix*., Junpei Enami, Naohito Kondo, Toshikazu Takano, Kaneo Suzuki., US 5264359., 1989).

Un deuxième problème réside dans le manque de place à l'intérieur du gel pour la croissance des cellules. Différentes stratégies ont donc été testées quant à la formation des capsules (*Encapsulation of biological material, Franklin Lim,* US 4352883, 1982*, Tissue culture and production in permeable gels.*, Elizabeth Maureen Frye, Mark Maurice Lynch, John Paul Vasington., EP0185701., 1984).

La demande de brevet WO 2010/063937 décrit un procédé pour la préparation de capsules présentant un coeur liquide et une enveloppe gélifiée de faible épaisseur encapsulant totalement le coeur. Ces capsules sont formées par co-extrusion de gouttes à la sortie d'une double enveloppe. Lesdites capsules sont décrites comme susceptibles de contenir des cellules. Néanmoins, même si le procédé semble adapté pour l'encapsulation cellulaire, rien n'indique que les capsules permettent de supporter la croissance cellulaire lorsque les cellules encapsulées sont cultivées, ni surtout l'amplification et différenciation de cellules à potentialité hématopoïétique, en particulier de cellules souches hématopoïétiques ou de cellules progénitrices érythroïdes.

Les inventeurs ont démontré que les capsules contenant des cellules à potentialité hématopoïétique selon l'invention permettent de solutionner tous ces problèmes et permettent donc d'envisager pour la première fois une application à la culture cellulaire de cellules à potentialité hématopoïétique à grande échelle.

Le but de l'invention est ainsi d'améliorer le rendement de cultures cellulaires de cellules à potentialité hématopoïétique, pour produire des cellules érythroïdes énucléées, dans un bioréacteur. L'enjeu est d'amener les nutriments et d'éliminer les catabolites nécessaires à la bonne croissance des cellules à potentialité hématopoïétique.

La capsule selon l'invention permet de protéger lesdites cellules du cisaillement ou des bulles d'oxygène en solution, tout en garantissant des conditions homogènes de croissance.

Avec une paroi poreuse aux petites molécules et une taille inférieure au millimètre, la diffusion suffit en effet à garantir l'homogénéité en concentration à l'intérieur des capsules. La paroi protégeant les cellules, il sera possible d'agiter une grande concentration de capsules efficacement et à grande échelle dans le bioréacteur. L'encapsulation présente d'autres avantages pour la culture de cellules à potentialité hématopoïétique à grande échelle: les capsules sont simples à manipuler (par sédimentation, capture au travers d'un filtre) et vont permettre par exemple de changer facilement le milieu de culture sans appliquer de stress sur les cellules, contrairement à un réacteur classique.

Ainsi, la présente invention concerne une capsule comprenant un coeur liquide, une enveloppe gélifiée encapsulant totalement le coeur liquide à sa périphérie, l'enveloppe gélifiée étant propre à retenir le coeur liquide lorsque la capsule est plongée dans un gaz, l'enveloppe gélifiée comprenant au moins un polyélectrolyte gélifié et au moins un agent tensioactif, dont le coeur liquide comprend au moins une cellule à potentialité hématopoïétique.

Par « cellules à potentialité hématopoïétique » on entend des cellules capables de se différencier vers une ou plusieurs des lignées qui sont à l'origine des cellules sanguines.

Plus particulièrement, les « cellules à potentialité hématopoïétique » selon l'invention sont des cellules de la lignée érythroïde, susceptibles de produire des globules rouges lors de leur différenciation. Les cellules à potentialité hématopoïétique selon l'invention peuvent notamment provenir des cellules souches, en particulier des cellules souches embryonnaires (ESC), des cellules souches adultes, comme les cellules souches hématopoïétiques (CSH), des cellules souches induites pluripotentes (iPS), de cellules immortalisées, de cellules progénitrices érythroïdes ou de précurseurs érythroïdes. De préférence, les « cellules à potentialité hématopoïétique » de l'invention sont des cellules humaines.

De préférence, les cellules à potentialité hématopoïétique sont des cellules souches hématopoïétiques et/ou des cellules progénitrices érythroïdes et/ou des précurseurs érythroïdes.

Lorsque les cellules à potentialité hématopoïétique sont des cellules souches hématopoïétiques, celles-ci sont de préférence des cellules souches hématopoïétiques humaines, en particulier des cellules CD34+ qui peuvent être obtenues à partir de sang de cordon ombilical ou par leucophérèse, à partir de sang périphérique. Elles se différencieront préférentiellement en réticulocytes et/ou érythrocytes.

Les érythrocytes, ou hématies, sont plus communément appelés globules rouges.

Les réticulocytes sont les cellules précédant le stade d'érythrocyte dans l'érythropoïèse. Ils leur sont quasiment semblables. Les réticulocytes sont des globules rouges jeunes qui possèdent encore des ribosomes et des mitochondries, mais sont dépourvus de péroxysome.

Par « cellule progénitrice érythroïde », on entend une cellule issue de la différentiation d'une cellule souche hématopoïétique, capable de proliférer et de se différencier en cellule de la lignée érythroïde, en particulier en réticulocytes et/ou érythrocytes. Elles sont de préférence des cellules progénitrices érythroïdes humaines.

Par «précurseur érythroïdes », on entend toute cellule de la lignée érythroïde, cytologiquement identifiable, c'est à dire répondant aux critères classiques d'identification des types cellulaires allant du proérythroblaste à l'érythroblaste acidophile. Ils sont de préférence des précurseurs érythroïdes humains.

Dans le cadre de la présente invention, le coeur liquide de la capsule peut comprendre au moment de l'encapsulation une quantité de cellules à potentialité hématopoïétique comprise entre 1 et 10000, de préférence entre 10 et 1000 cellules/capsule.

Le coeur liquide de la capsule selon l'invention est constitué d'un liquide de préférence physiologiquement acceptable, comme une solution saline, une solution tampon, un viscosifiant physiologiquement acceptable et/ou un milieu de culture destiné à la croissance et à la différenciation des cellules à potentialité hématopoïétique, dont la composition sera détaillée dans la suite de la description.

Le coeur liquide peut aussi comprendre des excipients physiologiquement acceptables, tels que des épaississants, ou des modificateurs de rhéologie. Ces épaississants sont par exemple des polymères, des cross-polymères, des microgels, des gommes ou des protéines, dont des polysaccharides, des celluloses, des polyosides, des polymères et co-polymères à base de silicone, des particules colloïdales (silice, argiles, latex...).

L'enveloppe gélifiée des capsules selon l'invention comprend un gel contenant de l'eau et au moins un polyélectrolyte réactif aux ions multivalents. Selon l'invention, l'enveloppe contient en outre un agent tensioactif résultant de son procédé de fabrication, comme décrit par la suite.

En particulier, la capsule selon l'invention est obtenue à partir d'un procédé comprenant les étapes suivantes de :
a) convoyage séparé dans une double enveloppe d'une première solution liquide contenant au moins une cellule à potentialité hématopoïétique et d'une deuxième solution liquide contenant un polyélectrolyte liquide propre à gélifier;
b) formation, à la sortie de la double enveloppe, d'une série de gouttes, chaque goutte comprenant un noyau central formé de ladite première solution et une pellicule périphérique formée de ladite deuxième solution et recouvrant totalement le noyau central;
c) immersion de chaque goutte dans une solution gélifiante contenant un réactif propre à réagir avec le polyélectrolyte de la pellicule pour le faire passer d'un état liquide à un état gélifié et former l'enveloppe gélifiée, le noyau central formant le coeur liquide;
d) récupération des capsules formées;
   la deuxième solution contenant au moins un agent tensioactif avant son contact avec la première solution.

Selon un des aspects de ce procédé, le rapport du débit de la première solution au débit de la deuxième solution à la sortie de la double enveloppe est compris entre 1 et 200, avantageusement entre 10 et 200, l'enveloppe gélifiée présentant une épaisseur comprise entre 0,1% et 10%, avantageusement entre 0,1% et 2% du diamètre de la capsule, après récupération des capsules formées.

Selon un autre aspect du procédé, la première solution liquide physiologiquement acceptable comprend une solution saline, une solution tampon, un viscosifiant physiologiquement acceptable, un excipient physiologiquement acceptable, avantageusement un épaississant ou un modificateur de rhéologie, et/ou du milieu de culture.

Selon un autre aspect du procédé, les gouttes formées par coextrusion dans la double enveloppe tombent par gravité à travers un volume d'air dans la solution gélifiante.

Dans le cadre de la présente description, on entend par « tensioactif » une molécule amphiphile présentant deux parties de polarité différente, l'une lipophile et apolaire, l'autre hydrophile et polaire. Un tensioactif peut être de type ionique (cationique ou anionique), zwitterionique ou non ionique.

L'agent tensioactif est avantageusement un tensioactif anionique, un tensioactif non ionique, un tensioactif cationique ou un mélange de ceux-ci. La masse moléculaire de l'agent tensioactif est comprise entre 150 g/mol et 10000 g/mol, avantageusement entre 250 g/mol et 1500 g/mol.

Dans le cas où le tensioactif est un tensioactif anionique, il est par exemple choisi parmi un alkylsulfate, un alkyle sulfonate, un alkylarylsulfonate, un alkylphosphate alcalin, un dialkylsulfosuccinate, un sel d'alcalino-terreux d'acides gras saturés ou non. Ces tensioactifs présentent avantageusement au moins une chaîne hydrocarbonée hydrophobe présentant un nombre de carbones supérieur à 5, voire 10 et au moins un groupement anionique hydrophile, tel qu'un sulfate, un sulfonate ou un carboxylate lié à une extrémité de la chaîne hydrophobe.

Dans le cas où le tensioactif est un tensioactif cationique, il est par exemple choisi parmi un sel d'halogénure d'alkylpyridium ou d'alkylammonium comme le chlorure ou le bromure de n-éthyldodecylammonium, le chlorure ou le bromure de cétylammonium (CTAB). Ces tensioactifs présentent avantageusement au moins une chaîne hydrocarbonée hydrophobe présentant un nombre de carbones supérieur à 5, voire 10 et au moins un groupement cationique hydrophile, tel qu'un cation d'ammonium quaternaire.

Dans le cas où le tensioactif est un tensioactif non ionique, il est par exemple choisi parmi des dérivés polyoxyéthylénés et/ou polyoxypropylénés des alcools gras, des acides gras, ou des alkylphénols, des arylphénols, ou parmi des alkyls glucosides, des polysorbates, des cocamides.

En particulier, l'agent tensioactif sera choisi dans la liste suivante : un alkylsulfate, un alkyle sulfonate, un alkylarylsulfonate, un alkylphosphate alcalin, un dialkylsulfosuccinate, un sel d'alcalino-terreux d'acides gras saturés ou non, un sel d'halogénure d'alkylpyridium ou d'alkylammonium comme le chlorure ou le bromure de n-éthyldodecylammonium, le chlorure ou le bromure de cétylamonium, des dérivés polyoxyéthylénés et/ou polyoxypropylénés des alcools gras, des acides gras ou des alkylphénols, ou parmi des arylphénols, des alkyls glucosides, des polysorbates, des cocamides ou leurs mélanges.

Plus particulièrement, le pourcentage massique total en agent tensioactif dans la deuxième solution sera supérieur à 0,01% et est avantageusement compris entre 0,01% et 0,5% en masse.

Par « polyélectrolyte réactif aux ions polyvalents », on entend, au sens de la présente invention, un polyélectrolyte susceptible de passer d'un état liquide dans une solution aqueuse à un état gélifié sous l'effet d'un contact avec une solution gélifiante contenant des ions multivalents tels que des ions d'un métal alcalino-terreux choisis par exemple parmi les ions calcium, les ions baryum, les ions magnésium.

Dans l'état liquide, les chaînes individuelles de polyélectrolyte sont sensiblement libres de s'écouler les unes par rapport aux autres. Une solution aqueuse de 2% en masse de polyélectrolyte présente alors un comportement purement visqueux aux gradients de cisaillement caractéristiques du procédé de mise en forme. La viscosité de cette solution à cisaillement nul est entre 50 mPa.s et 10000 mPa.s, avantageusement entre 3000 mPa.s et 7000 mPa.s.

Les chaînes individuelles de polyélectrolyte dans l'état liquide présentent avantageusement une masse molaire supérieure à 65000 g/mole.

Dans l'état gélifié, les chaînes individuelles de polyélectrolyte forment, avec les ions multivalents, un réseau tridimensionnel cohérent qui retient le coeur liquide et empêche son écoulement. Les chaînes individuelles sont retenues les unes par rapport aux autres et ne peuvent pas s'écouler librement les unes par rapport aux autres. Dans cet état, la viscosité du gel formé est infinie. De plus, le gel a un seuil de contrainte à l'écoulement. Ce seuil de contrainte est supérieur à 0,05 Pa. Le gel possède également un module d'élasticité non nul et supérieur à 35 kPa.

Le gel tridimensionnel de polyélectrolyte contenu dans l'enveloppe emprisonne de l'eau et l'agent tensioactif.

Le polyélectrolyte est de préférence un polymère biocompatible inoffensif pour le corps humain. Il est par exemple produit biologiquement.

Avantageusement, il est choisi parmi les polysaccharides, polyélectrolytes de synthèse à base d'acrylates (polyacrylate de sodium, de lithium, de potassium ou d'ammonium, ou polyacrylamide), de polyélectrolytes de synthèse à base de sulfonates (poly(styrène sulfonate) de sodium, par exemple). Plus particulièrement, le polyélectrolyte est choisi parmi un alginate d'alcalino-terreux, tel qu'un alginate de sodium ou un alginate de potassium, une gellane ou une pectine.

Les alginates sont produits à partir d'algues brunes appelées «laminaires», désignées par le terme anglais « sea weed ».

De tels alginates présentent avantageusement une teneur en α-L-guluronate supérieure à environ 50%, de préférence supérieure à 55%, voire supérieure à 60%.

En particulier, le ou chaque poyélectrolyte sera un polyélectrolyte réactif aux ions multivalents, notamment un polysaccharide réactif aux ions multivalents tel qu'un alginate d'alcalin, une géllane ou une pectine, de préférence un alginate d'alcalin ayant avantageusement une teneur en bloc α-L-guluronate supérieure à 50%, notamment supérieure à 55%.

Plus particulièrement, la teneur massique en polyélectrolyte dans la deuxième solution peut être inférieure à 5% en masse et est avantageusement comprise entre 0,5 et 3% en masse.

Selon un aspect de la présente invention, la capsule pourra comprendre en outre une enveloppe intermédiaire encapsulant totalement à sa périphérie le coeur liquide, ladite enveloppe intermédiaire étant elle-même encapsulée totalement à sa périphérie par l'enveloppe gélifiée.

Cette enveloppe intermédiaire liquide sera formée d'une composition intermédiaire comprenant un tampon ou un milieu de culture cellulaire, et/ou un viscosifiant. En particulier, le viscosifiant sera un polymère hydrosoluble, tel que du PEG, du dextran ou encore de l'alginate en solution plus diluée que dans l'enveloppe extérieure.

L'enveloppe intermédiaire est au contact du coeur et de l'enveloppe externe et maintient le coeur hors de contact de l'enveloppe externe.

La phase intermédiaire est utile pour stabiliser la capsule lors de sa formation, par exemple dans le cas où le coeur liquide contient du calcium susceptible d'induire trop tôt la gélification de la phase externe. En effet, selon leur composition, les milieux de culture cellulaire peuvent interférer avec la polymérisation. Elle permet ainsi de séparer le coeur liquide de la phase externe à gélifier. Elle permet aussi de protéger le coeur liquide contenant les cellules pendant la formation des gouttes, de l'alginate de la couche externe qui n'est pas encore polymérisé.

En particulier, le coeur liquide et la phase intermédiaire étant toutes deux liquides, celles-ci se mélangent à terme pour former le coeur liquide de la capsule.

La présence d'une telle enveloppe intermédiaire est notamment décrite dans l'article scientifique « Formation of liquid-core capsules having a thin hydrogel membrane : liquid pearls », Bremond et al, Soft Matter, 2010, 2484-2488.

### Production des gouttes

La production des gouttes selon le procédé selon l'invention mentionné précédemment est effectuée par convoyage séparé dans une double enveloppe d'une première solution liquide contenant la ou les cellules à potentialité hématopoïétique et d'une deuxième solution liquide contenant un polyélectrolyte liquide propre à gélifier et au moins un agent tensioactif, tel que décrit dans WO2010/063937.

Dans le cas de la présence supplémentaire d'une enveloppe intermédiaire, le convoyage séparé s'effectue dans une triple enveloppe, avec une troisième solution comprenant la solution intermédiaire.

A la sortie de la double (ou triple) enveloppe, les différents flux entrent en contact et se forme alors une goutte multi-composante, selon un mode hydrodynamique dit de « dripping » (goutte-à-goutte, décrit notamment dans WO 2010/063937) ou un mode dit de « jetting » (avec une instabilité de jet, décrit notamment dans FR 2012/2964017), selon la taille des capsules souhaitées.

Le premier flux constitue le coeur liquide et le deuxième flux constitue l'enveloppe externe liquide. Dans le cas de la présence de l'enveloppe intermédiaire, le deuxième flux constitue l'enveloppe intermédiaire liquide et le troisième flux l'enveloppe externe liquide.

Selon le mode de production, chaque goutte multi-composante se détache de la double (ou triple) enveloppe et chute dans un volume d'air, avant d'être immergée dans une solution gélifiante contenant un réactif propre à gélifier le polyélectrolyte de l'enveloppe externe liquide, pour former l'enveloppe externe gélifiée des capsules selon l'invention.

Selon certaines variantes, les gouttes multi-composantes peuvent comprendre des couches supplémentaires entre l'enveloppe externe et le coeur liquide, autre que l'enveloppe intermédiaire. Ce type de goutte peut être préparé par convoyage séparé de multiples compositions dans des dispositifs à multiples enveloppes.

### Etape de gélification

Lorsque la goutte multi-composante entre en contact de la solution gélifiante, le réactif propre à gélifier le polyélectrolyte présent dans la solution gélifiante forme alors des liaisons entre les différentes chaînes de polyélectrolyte présentes dans l'enveloppe externe liquide, passant alors à l'état gélifié, provoquant ainsi la gélification de l'enveloppe externe liquide.

Sans vouloir être lié à une théorie particulière, lors du passage à l'état gélifié du polyélectrolyte, les chaînes individuelles de polyélectrolyte présentes dans l'enveloppe externe liquide se raccordent les unes aux autres pour former un réseau réticulé, aussi appelé hydrogel.

Dans le cadre de la présente description, le polyélectrolyte présent dans l'enveloppe externe gélifiée est à l'état gélifié et est également appelé polyélectrolyte à l'état gélifié ou encore polyélectrolyte gélifié.

Une enveloppe externe gélifiée, propre à retenir l'ensemble constitué par le coeur ou le coeur et l'enveloppe intermédiaire, est ainsi formée. Cette enveloppe externe gélifiée présente une tenue mécanique propre, c'est-à-dire qu'elle est capable retenir le coeur liquide et, en cas de présence d'une enveloppe intermédiaire, d'entourer totalement l'enveloppe intermédiaire. Ceci a pour effet de maintenir la structure interne du coeur liquide et le cas échéant, de l'enveloppe intermédiaire.

Les capsules selon l'invention séjournent dans la solution gélifiante le temps que l'enveloppe externe soit complètement gélifiée, de préférence sans excéder 30 minutes, encore plus préférentiellement sans excéder 5 minutes.

On peut ensuite éventuellement éliminer la solution gélifiante et les capsules gélifiées peuvent ensuite éventuellement être collectées et plongées dans une solution aqueuse de rinçage, généralement essentiellement constituée d'eau, en particulier de l'eau physiologique, afin de rincer les capsules gélifiées formées. Cette étape de rinçage permet d'extraire de l'enveloppe externe gélifiée un éventuel excès du réactif propre à gélifier de la solution gélifiante, et tout ou partie du tensioactif (ou d'autres espèces) contenu initialement dans la deuxième solution liquide.

La présence d'un tensioactif dans la deuxième solution liquide permet d'améliorer la formation et la gélification des gouttes multi-composantes selon le procédé tel que décrit précédemment.

### Caractéristiques des capsules gélifiées

Avantageusement, la capsule est de forme sphérique et présente un diamètre extérieur inférieur à 5 mm et compris notamment entre 0.3 mm et 3 mm.

De préférence, l'enveloppe externe gélifiée des capsules selon l'invention possède une épaisseur comprise de 10 µm à 500 µm, de préférence de 20 µm à 200 µm, et avantageusement de 50 µm à 100 µm.

La finesse de l'épaisseur de l'enveloppe externe gélifiée permet généralement de rendre cette enveloppe externe transparente.

Les capsules selon l'invention présentent généralement un rapport volumique entre le coeur et l'ensemble des enveloppes intermédiaire et externe supérieur à 2, et de préférence inférieur à 50.

Selon un mode de réalisation particulier, les capsules selon l'invention présentent généralement un rapport volumique entre le coeur et l'ensemble des enveloppes intermédiaire et externe compris entre 5 et 10.

L'invention concerne également l'utilisation d'au moins une capsule telle que décrite dans le cadre de la présente invention, pour la production *ex vivo* de cellules érythroïdes énucléées, en particulier de réticulocytes et/ou d'érythrocytes.

Elle concerne aussi une méthode *ex vivo* de production de cellules érythroïdes énucléées comprenant la culture de cellules à potentialité hématopoïétique contenues dans au moins une capsule telle que définie dans le cadre de la présente invention, dans des conditions permettant la production de cellules érythroïdes énucléées.

De façon particulière, les cellules érythroïdes énucléées produites selon la méthode de la présente invention sont des réticulocytes et/ou des érythrocytes.

En particulier, les cellules à potentialité hématopoïétique utilisées dans le cadre de la méthode selon l'invention sont des cellules souches hématopoïétiques et/ou des cellules progénitrices érythroïdes et/ou des précurseurs érythroïdes, plus particulièrement humains.

Ainsi, les cellules à potentialité hématopoïétique peuvent être cultivées dans le cadre de la présente invention dans un milieu de culture comprenant :
a) de l'insuline à une concentration comprise entre 1 et 50 µg/ml;
b) de la transferrine à une concentration comprise entre 100 et 2000 µg/ml; et
c) du plasma ou du sérum à une concentration comprise entre 1 et 30%.

Par « milieu de culture », on entend tout milieu, en particulier tout milieu liquide, susceptible de soutenir la croissance des cellules à potentialité hématopoïétique, en particulier des cellules souches hématopoïétiques, des cellules progénitrices érythroïdes et des précurseurs érythroïdes, plus particulièrement humains, et de permettre la production de cellules érythroïdes énucléées, en particulier de réticulocytes et/ou d'érythrocytes.

L'insuline du milieu de culture selon la présente invention est, en particulier, de l'insuline recombinante humaine. Sa concentration est préférentiellement comprise entre 5 et 20 µg/ml, plus préférentiellement entre 8 et 12 µg/ml, et encore plus préférentiellement de 10 µg/ml.

La transferrine du milieu de culture selon l'invention est, en particulier, de la transferrine humaine. Plus particulièrement, la transferrine est saturée en fer. Sa concentration est préférentiellement comprise entre 200 et 1000 µg/ml, plus préférentiellement entre 300 et 500 µg/ml, et encore plus préférentiellement de 330 ou 450 µg/ml. La transferrine peut également se présenter sous forme recombinante.

Le plasma ou le sérum du milieu de culture selon l'invention sont, en particulier, humains. Leur concentration est préférentiellement comprise entre 1 et 20%, plus préférentiellement entre 4 et 12%, et encore plus préférentiellement de 5 ou 10%.

Selon un aspect de la présente invention, le milieu de culture comprend également de l'héparine, en particulier à une concentration comprise entre 0,5 et 5 UI/ml, préférentiellement entre 1,5 et 3,5 Ul/ml, et encore plus préférentiellement de 2 UI/ml. En particulier, le milieu de culture selon la présente invention comprend de l'héparine quand le milieu de culture comprend également du plasma.

Le milieu de culture peut également comprendre de l'EPO et/ou du SCF et/ou de l'IL-3 et/ou de l'hydrocortisone.

L'EPO (érythropoïétine) du milieu de culture selon l'invention est, en particulier, de l'EPO humaine recombinante. Sa concentration est préférentiellement comprise entre 0,5 et 20 Ul/ml, plus préférentiellement entre 2,5 et 3,5 Ul/ml, et encore plus préférentiellement de 3 UI/ml.

Le SCF (Stem Cell Factor) du milieu de culture selon l'invention est, en particulier, du SCF humain recombinant. Sa concentration est préférentiellement comprise entre 50 et 200 ng/ml, plus préférentiellement entre 80 et 120 ng/ml, et encore plus préférentiellement de 100 ng/ml.

L'IL-3 (interleukine 3) du milieu de culture selon l'invention est, en particulier, de l'IL-3 humaine recombinante. Sa concentration est préférentiellement comprise entre 1 et 30 ng/ml, plus préférentiellement entre 4 et 6 ng/ml, et encore plus préférentiellement de 5 ng/ml.

L'hydrocortisone, qui est ajouté optionnellement dans le milieu de culture, a selon l'invention préférentiellement une concentration comprise entre 5.10⁻⁷ et 5.10⁻⁶ M, et plus préférentiellement de 5.10⁻⁶ M.

Selon un aspect de la présente invention, le milieu de culture peut comprendre également au moins un des composés suivants : TPO, FLT3, BMP4, VEGF-A165 et IL-6.

La TPO (thrombopoïétine) du milieu de culture selon l'invention est, en particulier, de la TPO humaine recombinante. Sa concentration est préférentiellement comprise entre 20 et 200 ng/ml, plus préférentiellement entre 80 et 120 ng/ml, et encore plus préférentiellement de 100 ng/ml.

Le FLT3 (FMS-like tyrosine kinase 3 ligand) du milieu de culture selon l'invention est, en particulier, du FLT3 humain recombinant. Sa concentration est préférentiellement comprise entre 20 et 200 ng/ml, plus préférentiellement entre 80 et 120 ng/ml, et encore plus préférentiellement de 100 ng/ml.

La BMP4 (Bone Morphogenic Protein 4) du milieu de culture selon l'invention est, en particulier, de la BMP4 humaine recombinante. Sa concentration est préférentiellement comprise entre 1 et 20 ng/ml, plus préférentiellement entre 8 et 12 ng/ml, et encore plus préférentiellement de 10 ng/ml.

Le VEGF-A165 (Vascular Endothelial Growth Factor A165) du milieu de culture selon l'invention est, en particulier, du VEGF-A165 humain recombinant. Sa concentration est préférentiellement comprise entre 1 et 20 ng/ml, plus préférentiellement entre 4 et 6 ng/ml, et encore plus préférentiellement de 5 ng/ml.

L'IL-6 (interleukine 6) du milieu de culture selon l'invention est, en particulier, de l'IL-6 humaine recombinante. Sa concentration est préférentiellement comprise entre 1 et 20 ng/ml, plus préférentiellement entre 4 et 6 ng/ml, et encore plus préférentiellement de 5 ng/ml.

Dans le cadre de la présente invention, le milieu de culture comprend un milieu de culture de base, celui-ci ayant pour caractéristique d'être capable de soutenir la croissance des cellules à potentialité hématopoïétique, en particulier des cellules souches hématopoïétiques, des cellules progénitrices érythroïdes et/ou des précurseurs érythroïdes, plus particulièrement humains, et de permettre la production de cellules érythroïdes énucléés, en particulier de réticulocytes et/ou d'érythrocytes. Ce type de milieu de culture de base est bien connu de l'homme du métier. On peut citer par exemple le milieu Iscove Dulbecco modifié (IMDM), complété par de la glutamine ou un peptide contenant de la glutamine.

Ainsi le milieu de culture selon la présente invention comprend également préférentiellement du milieu Iscove Dulbecco modifié, complété par de la glutamine ou un peptide contenant de la glutamine.

En particulier, les cellules à potentialité hématopoïétique sont cultivées dans un milieu comprenant :

### lors d'une première étape de 5 à 9 jours, en particulier 7 jours:

- de l'insuline à une concentration comprise entre 8 et 12 µg/ml;
- de la transferrine à une concentration comprise entre 300 et 350 µg/ml;
- du plasma à une concentration comprise entre 3 et 7% ;
- de l'héparine à une concentration comprise entre 1,5 et 2,5 UI/ml ;
- optionnellement, de l'hydrocortisone à une concentration comprise entre 5.10⁻⁷ et 5.10⁻⁶ M;
- du SCF à une concentration comprise entre 80 et 120 ng/ml ;
- de l'IL-3 à une concentration comprise entre 4 et 6 ng/ml ; et
- de l'EPO à une concentration comprise entre 2,5 et 3,5 IU/ml ;

### puis lors d'une seconde étape de 0 à 5 jours, en particulier de 3 à 4 cours:

- de l'insuline à une concentration comprise entre 8 et 12 µg/ml;
- de la transferrine à une concentration comprise entre 300 et 350 µg/ml;
- du plasma à une concentration comprise entre 3 et 7% ;
- de l'héparine à une concentration comprise entre 1,5 et 2,5 UI/ml ;
- optionnellement de l'hydrocortisone à une concentration comprise entre 5.10⁻⁷ et 5.10⁻⁶ M ;
- du SCF à une concentration comprise entre 80 et 120 ng/ml ; et
- de l'EPO à une concentration comprise entre 2,5 et 3,5 IU/ml ;

### et dans une troisième étape de 6 à 10 jours, en particulier jusqu'à 18 ou 21 jours depuis le début de la première étape:

- de l'insuline à une concentration comprise entre 8 et 12 µg/ml;
- de la transferrine à une concentration comprise entre 300 et 350 µg/ml;
- du plasma à une concentration comprise entre 3 et 7% ;
- de l'héparine à une concentration comprise entre 1,5 et 2,5 Ul/ml ; et
- de l'EPO à une concentration comprise entre 2,5 et 3,5 IU/ml.

Alternativement, les cellules à potentialité hématopoïétique sont cultivées dans un milieu comprenant :

### lors d'une première étape de 6 à 8 jours, en particulier 7 cours:

- de l'insuline à une concentration comprise entre 8 et 12 µg/ml;
- de la transferrine à une concentration comprise entre 300 et 350 µg/ml;
- du plasma à une concentration comprise entre 1% et 7% ;
- de l'héparine à une concentration comprise entre 1,5 et 2,5 UI/ml ;
- du SCF à une concentration comprise entre 80 et 120 ng/ml ;
- de l'IL-3 à une concentration comprise entre 5 et 30 ng/ml ;
- de la TPO à une concentration comprise entre 80 et 120 ng/ml ; et
- du FLT3 à une concentration comprise entre 30 et 60 ng/ml ;

### puis lors d'une seconde étape de 10 à 16 jours, en particulier de 14 cours:

- de l'insuline à une concentration comprise entre 8 et 12 µg/ml;
- de la transferrine à une concentration comprise entre 300 et 350 µg/ml;
- du plasma à une concentration comprise entre 1% et 7% ;
- de l'héparine à une concentration comprise entre 1,5 et 2,5 UI/ml ;
- optionnellement de l'hydrocortisone à une concentration comprise entre 5.10⁻⁷ et 5.10⁻⁶ M ;
- du SCF à une concentration comprise entre 80 et 120 ng/ml ;
- de l'EPO à une concentration comprise entre 2,5 et 3,5 IU/ml ; et
- de l'IL-3 à une concentration comprise entre 4 et 6 ng/ml ;

### et dans une troisième étape de 6 à 12 jours, en particulier jusqu'à 28 ou 32 jours depuis le début de la première étape :

- de l'insuline à une concentration comprise entre 8 et 12 µg/ml ;
- de la transferrine à une concentration comprise entre 300 et 350 µg/ml ;
- du plasma à une concentration comprise entre 1% et 7% ;
- de l'héparine à une concentration comprise entre 1,5 et 2,5 Ul/ml ; et
- de l'EPO à une concentration comprise entre 2,5 et 3,5 IU/ml.

Alternativement, les cellules à potentialité hématopoïétique sont cultivées dans un milieu comprenant :

### lors d'une première étape de 5 à 25 jours, en particulier 20 jours :

- de l'insuline à une concentration comprise entre 8 et 12 µg/ml ;
- de la transferrine à une concentration comprise entre 425 et 475 µg/ml ;
- du plasma à une concentration comprise entre 3 et 7% ;
- de l'héparine à une concentration comprise entre 1,5 et 2,5 UI/ml ;
- du SCF à une concentration comprise entre 80 et 120 ng/ml ;
- de la TPO à une concentration comprise entre 80 et 120 ng/ml ;
- du FLT3 à une concentration comprise entre 80 et 120 ng/ml ;
- de la BPM4 à une concentration comprise entre 8 et 12 ng/ml ;
- du VEGF-A165 à une concentration comprise entre 4 et 6 ng/ml.
- de l'IL-3 à une concentration comprise entre 4 et 6 ng/ml ;
- de l'IL-6 à une concentration comprise entre 4 et 6 ng/ml ; et
- de l'EPO à une concentration comprise entre 2,5 et 3,5 IU/ml ;

### puis lors d'une seconde étape de 6 à 10 jours, en particulier 8 jours :

- de l'insuline à une concentration comprise entre 8 et 12 µg/ml ;
- de la transferrine à une concentration comprise entre 425 et 475 µg/ml ;
- du plasma à une concentration comprise entre 8 et 12% ;
- de l'héparine à une concentration comprise entre 2,5 et 3,5 UI/ml ;
- du SCF à une concentration comprise entre 80 et 120 ng/ml ;
- de l'IL-3 à une concentration comprise entre 4 et 6 ng/ml ; et
- de l'EPO à une concentration comprise entre 2,5 et 3,5 IU/ml ;

### puis lors d'une troisième étape de 2 à 4 jours, en particulier 3 jours :

- de l'insuline à une concentration comprise entre 8 et 12 µg/ml ;
- de la transferrine à une concentration comprise entre 425 et 475 µg/ml ;
- du plasma à une concentration comprise entre 8 et 12% ;
- de l'héparine à une concentration comprise entre 2,5 et 3,5 UI/ml ;
- du SCF à une concentration comprise entre 80 et 120 ng/ml ; et
- de l'EPO à une concentration comprise entre 2,5 et 3,5 IU/ml ;

### et dans une quatrième étape de 10 à 16 jours, en particulier 13 jours :

- de l'insuline à une concentration comprise entre 8 et 12 µg/ml ;
- de la transferrine à une concentration comprise entre 425 et 475 µg/ml ;
- du plasma à une concentration comprise entre 8 et 12% ;
- de l'héparine à une concentration comprise entre 2,5 et 3,5 UI/ml; et
- de l'EPO à une concentration comprise entre 2,5 et 3,5 IU/ml.

Dans le cadre de la présente invention, les cellules sont de préférence encapsulées à J0 c'est-à-dire à un stade de différentiation très peu avancé, par exemple à celui de cellules souches hématopoïétiques, mais peuvent également être encapsulées à un stade ultérieur, c'est-à-dire au stade de progéniteur ou de précurseur érythroïde.

Les capsules selon l'invention comprenant au moins une cellule à potentialité hématopoïétique, pourront par conséquent comprendre uniquement des cellules peu différenciées comme des cellules souches hématopoïétiques, uniquement des cellules à un stade de différentiation plus avancé tel que des cellules progénitrices érythroïdes, des précurseurs érythroïdes ou un mélange de cellules à potentialité hématopoïétique à différents stades de différentiation.

La présente invention sera illustrée plus en détail par les figures et exemples ci-dessous qui n'en limitent pas la portée.

### FIGURES

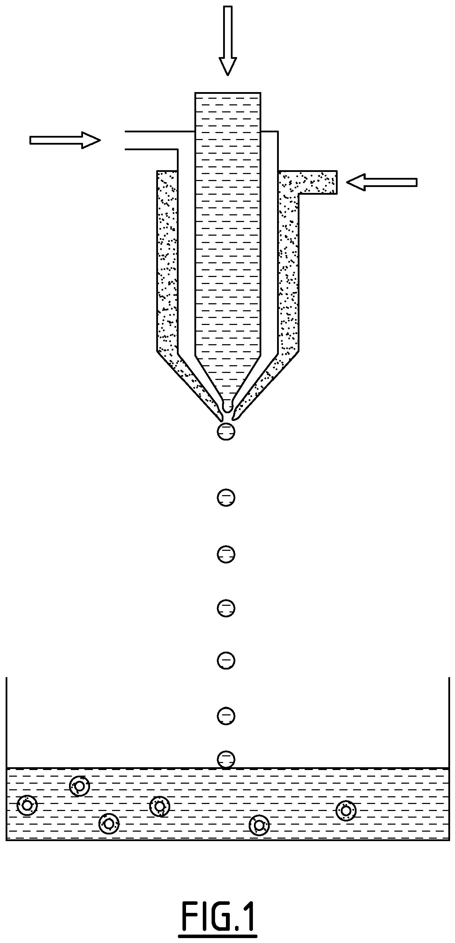

**Figure 1** **:** Principe de la préparation des capsules :
   Une structure en triple enveloppe est représentée dans laquelle le fluide constituant le coeur (qui comprend au moins une cellule à potentialité hématopoïétique) est introduit dans l'enveloppe centrale indiquée par la flèche verticale tandis que le fluide intermédiaire est introduit dans l'enveloppe intermédiaire indiquée par la flèche située à gauche de la structure en triple enveloppe et le fluide de la coque dans l'enveloppe extérieure indiquée par la flèche située à droite de la structure en triple enveloppe.
      Les capsules ainsi formées tombent dans le bain de gélification.
**Figure 2** **:** Vue en perspective de l'injecteur.
**Figure 3** **:** Vue en coupe de l'injecteur :
   L'injecteur comprend trois entrées, la première entrée située sur la gauche de la vue en coupe correspondant à l'entrée du fluide intermédiaire, la deuxième entrée correspondant à la première entrée située sur le dessus de la vue en coupe correspondant à l'entrée du fluide de coeur et la troisième entrée correspondant à la deuxième entrée située sur le dessus de la vue en coupe correspondant à l'entrée du fluide de coque.

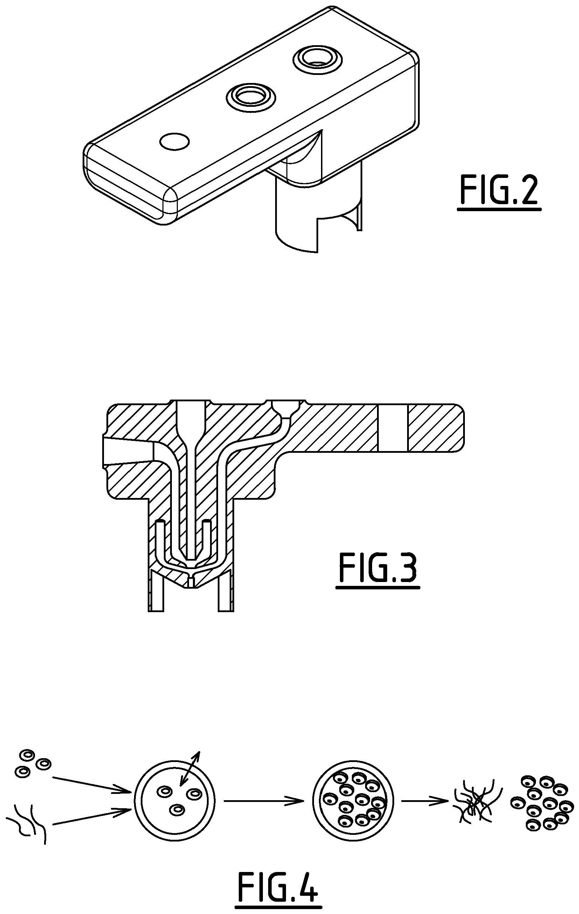

**Figure 4** **:** Encapsulation des cellules.

### EXEMPLE

### Culture de cellules à potentialité hématopoïétique encapsulées

### Préparation des cellules

Les cellules CD34+ sont isolées à partir de sang placentaire par sélection à l'aide de microbilles supermagnétiques en utilisant des colonnes Mini-MACS (Miltenyi Biotech, Bergisch Glodbach, Allemagne) (pureté supérieure à 94 ± 3%).

Les cellules sont cultivées dans du milieu IMDM (Iscove modified Dulbecco's, Biochrom, Allemagne) supplémenté avec 2 mM de L-glutamine (Invitrogen, Cergy-Pontoise, France), 330 µg/ml de transferrine humaine saturée en fer, 10 µg/ml d'insuline (Sigma, Saint-Quentin Fallavier, France), 2 IU/ml d'héparine Choay (Sanofi, France) et 5% de plasma (solvant/detergent virus inactivated plasma (S/D)). 10⁴ cellules CD34+/ml sont cultivées en présence de 100 ng/ml de SCF (fourni par Amgen, Thousand Oaks, CA), 5 ng/ml d'IL-3 (R&D Systems, Abingdon, Royaume-Uni.) et 3 IU/ml d'EPO (Eprex, fourni par Janssen Cilag, Issy-les-Moulineaux, France). Au jour 4, un volume de culture cellulaire est dilué dans quatre volumes de milieu frais contenant SCF, IL-3 et EPO. Les cultures sont maintenues à 37°C avec 5% de CO₂ dans l'air. Au jour 8, les cellules sont comptées et leur concentration est ajustée dans le milieu d'encapsulation (IMDM, héparine, plasma, insuline, transferrine, EPO, SCF et IL-3) afin d'atteindre la concentration voulue.

Les cellules sont encapsulées à J8.

### Protocole d'encapsulation des cellules

Le principe général de formation des capsules est indiqué en Figure 1.

La veille de la procédure d'encapsulation, les éléments suivants ont été préparés.

Un litre de CaCl₂ à 1% a été filtré sur un filtre de 0,2 µm pour servir au bain de gélification. Deux litres d'eau physiologique (NaCl 0,9%) sont préparés qui serviront à rincer le set up, c'est-à-dire l'injecteur, les tubes, les seringues et les connecteurs formant le dispositif d'encapsulation, ainsi que les capsules. Un litre d'eau milliQ est également préparé pour la mise en place du set up sous l'eau. Enfin, une solution de 30 ml d'alginate à 2% avec 0,5 mM de SDS est préparée. Les éléments suivants ont été autoclavés : cristallisoir, 10 béchers de 150ml (pour récupérer les capsules et les rincer), une pince, des connecteurs microfluidiques (pour le diamètre de tube 1/16 inch), des tubes en téflon (diamètre 1/16 inch ; 60 cm).

Le jour suivant, le dispositif d'encapsulation est monté selon le mode opératoire suivant: le set up est lavé à l'EtOH 70% puis à l'eau milliQ filtrée (injecteur XII voir Figures 2 et 3). Il comprend des capillaires dont le diamètre intérieur est de 0,78 mm et le diamètre extérieur de 1 mm ainsi que 3 seringues SGE (VWR), celle pour la phase interne de 5 ml avec agitation, celles pour les deux autres phases de 10 ml. Le set up est monté sous eau pour éviter la présence de bulles puis rempli avec du PBS/SVF (sérum de veau foetal) 10% et laissé pendant une heure afin d'éviter que les cellules n'adhèrent aux parois des tubes ou de l'injecteur par la suite. Il est ensuite rincé à l'eau physiologique. Le circuit de la phase intermédiaire est lavé avec de l'IMDM (Iscove's Modified Dulbecco's Medium) tandis que le circuit de la phase interne est lavé avec du milieu de culture contenant de l'IMDM, de l'héparine et du plasma. Le set up final est mis en place avec une solution d'alginate 2% pour la coque, de l'IMDM pour la phase intermédiaire et avec une suspension cellulaire comprenant entre 1,25.10⁵ cellules/ml et 2,5.10⁵ cellules/ml dans un milieu de culture complet contenant de l'IMDM, de l'héparine, du plasma, de l'insuline, de la transférine, de l'EPO, du SCF et de l'IL-3 pour la phase interne. L'encapsulation est réalisée comme indiqué en Figure 4 par un procédé d'encapsulation par goutte à goutte (dripping). Les débits utilisés pour les différentes phases interne-intermédiaire-coque sont respectivement de 5, 1 et 3 ml/h. Le temps de formation de la capsule est de 4,9 secondes. Les capsules ainsi obtenues font 1,6 mm de diamètre. Elles sont constituées d'une phase externe qui sera gélifiée en présence de calcium, d'une phase intermédiaire ayant pour but de stabiliser la capsule lors de sa formation, et d'une phase interne contenant les cellules.

L'étape de gélification est ensuite réalisée avec un bain de gélification contenant une solution de chlorure de calcium et une goutte de Tween 20 ® à 10%.

De 20 à 30 capsules sont plongées dans le bain de gélification. Les capsules contenant les cellules ne doivent pas rester dans le bain plus de cinq minutes.

La solution de calcium est ensuite éliminée sans faire tomber les capsules et celles-ci sont rincées avec 3x30 ml d'eau physiologique. L'eau physiologique est ensuite éliminée tandis que les capsules sont resuspendues dans 10 ml de milieu de culture. Le tout est ensuite transvasé dans un flacon de culture de 25 cm². Pour que les capsules soient bien immergées, les flacons sont mis à la verticale.

Les cellules sont ensuite cultivées dans du milieu frais en présence d'EPO jusqu'à J19. Les cultures sont maintenues à 37°C avec 5% de CO₂ dans l'air.

### Résultats

Les résultats sont indiqués dans le tableau 1 ci-dessous.

**Tableau 1**

| **nb cellules/capsule** [fraction volumique des cellules] | **Taux de multiplication J8 à J19** | **Taux d'énucléation** | **Taux de mortalité (Bleu Trypan)** | **Fraction volumique finale (J19)** |
|---|---|---|---|---|
| 850 [0,03%] | 705 | 42% | 8% | 2% |
| 850 [0,03%] | 579 | 62% | 8% | 2% |
| 1650 [0,06%] | 352 | 47% | 14% | 2,5% |
| 1650 [0,06%] | 354 | 61% | 15% | 2,5% |
| Culture classique (en flacon)[0,01%] | 585 | 70% | 10% | 0,1% |

Les cellules encapsulées présentent une coloration rouge, témoignant ainsi de la présence d'hémoglobine.

Les cellules encapsulées sont observées après dissolution de la capsule et coloration May-Grünwald Giemsa.

La présence de cellules mitotiques, de peu de cellules apoptotiques et de peu de cellules vacuolaires est observée.

Il est obtenu une fraction volumique de cellules de 2% dans les capsules après trois semaines de culture, et une viabilité équivalente à celle mesurée en culture statique.

Ainsi, les capsules contenant des cellules à potentialité hématopoïétique selon l'invention permettent d'envisager pour la première fois une application à la culture cellulaire de cellules à potentialité hématopoïétique à grande échelle.

## Revendications

1. Capsule comprenant un coeur liquide, une enveloppe gélifiée encapsulant totalement le coeur liquide à sa périphérie, l'enveloppe gélifiée étant propre à retenir le coeur liquide lorsque la capsule est plongée dans un gaz, l'enveloppe gélifiée comprenant au moins un polyélectrolyte gélifié et au moins un agent tensioactif, **caractérisée en ce que** le coeur liquide comprend au moins une cellule à potentialité hématopoïétique.

2. Capsule selon la revendication 1, **caractérisée en ce qu'**elle est obtenue par la mise en oeuvre d'un procédé comprenant les étapes suivantes de :
a) convoyage séparé dans une double enveloppe d'une première solution liquide physiologiquement acceptable contenant au moins une cellule à potentialité hématopoïétique et d'une deuxième solution liquide contenant un polyélectrolyte liquide propre à gélifier;
b) formation, à la sortie de la double enveloppe, d'une série de gouttes, chaque goutte comprenant un noyau central formé de ladite première solution et une pellicule périphérique formée de ladite deuxième solution et recouvrant totalement le noyau central;
c) immersion de chaque goutte dans une solution gélifiante contenant un réactif propre à réagir avec le polyélectrolyte de la pellicule pour le faire passer d'un état liquide à un état gélifié et former l'enveloppe gélifiée, le noyau central formant le coeur liquide;
d) récupération des capsules formées;
la deuxième solution contenant au moins un agent tensioactif avant son contact avec la première solution.

3. Capsule selon la revendication 2, **caractérisée en ce que** le rapport du débit de la première solution au débit de la deuxième solution à la sortie de la double enveloppe est compris entre 1 et 200, avantageusement entre 10 et 200, l'enveloppe gélifiée présentant une épaisseur comprise entre 0,1% et 10%, avantageusement entre 0,1% et 2% du diamètre de la capsule, après récupération des capsules formées.

4. Capsule selon l'une quelconque des revendications 2 ou 3, **caractérisée en ce que** les gouttes formées par coextrusion dans la double enveloppe tombent par gravité à travers un volume d'air dans la solution gélifiante.

5. Capsule selon l'une quelconque des revendications 2 à 4, **caractérisée en ce que** la première solution liquide physiologiquement acceptable comprend une solution saline, une solution tampon, un viscosifiant physiologiquement acceptable, un excipient physiologiquement acceptable, avantageusement un épaississant ou un modificateur de rhéologie, et/ou du milieu de culture.

6. Capsule selon l'une quelconque des revendications 1 à 5, **caractérisée en ce qu'**elle comprend en outre une enveloppe intermédiaire encapsulant totalement à sa périphérie le coeur liquide, ladite enveloppe intermédiaire étant elle-même encapsulée totalement à sa périphérie par l'enveloppe gélifiée.

7. Capsule selon l'une quelconque des revendications 1 à 6, **caractérisée en ce que** ladite au moins une cellule à potentialité hématopoïétique est une cellule souche hématopoïétique et/ou une cellule progénitrice érythroïde et/ou un précurseur érythroïde.

8. Capsule selon l'une quelconque des revendications 1 à 7, **caractérisée en ce que** ladite au moins une cellule à potentialité hématopoïétique ou cellule souche hématopoïétique ou cellule progénitrice érythroïde ou précurseur érythroïde est une cellule ou un précurseur humain.

9. Utilisation d'au moins une capsule selon l'une quelconque des revendications 1 à 8 pour la production *ex vivo* de cellules érythroïdes énucléées.

10. Utilisation selon la revendication 9, **caractérisée en ce que** les cellules érythroïdes énucléées sont des réticulocytes et/ou des érythrocytes.

11. Méthode *ex vivo* de production de cellules érythroïdes énucléées comprenant la culture de cellules à potentialité hématopoïétique contenues dans au moins une capsule telle que définie dans l'une quelconque des revendications 1 à 8, dans des conditions permettant la production de cellules érythroïdes énucléées.

12. Méthode selon la revendication 11, **caractérisée en ce que** les cellules à potentialité hématopoïétique sont cultivées dans un milieu de culture comprenant :
e) de l'insuline à une concentration comprise entre 1 et 50 µg/ml ;
f) de la transferrine à une concentration comprise entre 100 et 2000 µg/ml ; et
g) du plasma ou du sérum à une concentration comprise entre 1 et 30%.

13. Méthode selon la revendication 12, **caractérisée en ce que** le milieu de culture comprend également de l'EPO et/ou du SCF et/ou de l'IL-3 et/ou de l'hydrocortisone.

14. Méthode selon l'une quelconque des revendications 12 ou 13, **caractérisée en ce que** le milieu de culture comprend également au moins un des composés suivants : TPO, FLT3, BMP4, VEGF-A165 et IL-6.

15. Méthode selon l'une quelconque des revendications 12 à 14, **caractérisée en ce que** le milieu de culture comprend également du milieu Iscove Dulbecco modifié, complété par de la glutamine ou un peptide contenant de la glutamine.

16. Méthode selon l'une quelconque des revendications 11 à 15, **caractérisée en ce que** les cellules érythroïdes énucléées sont des réticulocytes et/ou des érythrocytes.

17. Méthode selon l'une quelconque des revendications 11 à 16, **caractérisée en ce que** les cellules à potentialité hématopoïétique sont des cellules souches hématopoïétiques et/ou des cellules progénitrices érythroïdes et/ou précurseurs érythroïdes.

## Patentansprüche

1. Kapsel, umfassend einen flüssigen Kern, eine gelierte Umhüllung, die vollständig den flüssigen Kern an seinem Umfang einkapselt, wobei die gelierte Umhüllung geeignet ist, den flüssigen Kern festzuhalten, wenn die Kapsel in ein Gas getaucht wird, und die gelierte Umhüllung mindestens ein geliertes Polyelektrolyt und mindestens ein Tensid umfasst, wobei der flüssige Kern mindestens eine Zelle mit hematopoetischem Potential aufweist.

2. Kapsel nach Anspruch 1, **dadurch gekennzeichnet, dass** sie durch Umsetzung eines Verfahrens erhalten wird, das die folgenden Schritte umfasst:
a) getrenntes Transportieren in einer doppelten Hülle einer ersten physiologisch verträglichen flüssigen Lösung, die mindestens eine Zelle mit hematopoetischem Potential enthält, und einer zweiten flüssigen Lösung, die ein flüssiges Polyelektrolyt enthält, das zum Gelieren geeignet ist;
b) Bilden einer Reihe von Tropfen am Ausgang der doppelten Hülle, wobei jeder Tropfen einen Mittelkern, der von der ersten Lösung gebildet wird, und einen umfänglichen Überzug, der von der zweiten Lösung gebildet wird und vollständig den Mittelkern bedeckt, umfasst;
c) Tauchen jedes Tropfens in eine Gelierlösung, die ein Reagenz enthält, das geeignet ist, mit dem Polyelektrolyt des Überzugs zu reagieren, um diesen von einem flüssigen Zustand in einen Gelierzustand zu bringen und die gelierte Umhüllung zu bilden, wobei der Mittelkern den flüssigen Kern bildet;
d) Rückgewinnen der gebildeten Kapseln;
wobei die zweite Lösung mindestens ein Tensid vor ihrem Kontakt mit der ersten Lösung enthält.

3. Kapsel nach Anspruch 2, **dadurch gekennzeichnet, dass** das Verhältnis der Durchflussmenge der ersten Lösung zur Durchflussmenge der zweiten Lösung am Ausgang der doppelten Hülle zwischen 1 und 200 liegt, vorzugsweise zwischen 10 und 200, wobei die gelierte Umhüllung eine Dicke zwischen 0,1% und 10%, vorzugsweise zwischen 0,1% und 2% des Durchmessers der Kapsel nach der Rückgewinnung der gebildeten Kapseln aufweist.

4. Kapsel nach einem beliebigen der Ansprüche 2 oder 3, **dadurch gekennzeichnet, dass** die durch Koextrusion in der doppelten Hülle gebildeten Tropfen mittels Schwerkraft durch ein Luftvolumen hindurch in die Gelierlösung fallen.

5. Kapsel nach einem beliebigen der Ansprüche 2 bis 4, **dadurch gekennzeichnet, dass** die erste physiologisch verträgliche flüssige Lösung eine Salzlösung, eine Pufferlösung, ein physiologisch verträgliches Andickungsmittel, einen physiologisch verträglicher Hilfsstoff, vorteilhafter Weise ein Verdickungsmittel oder einen rheologischen Modifikator und/oder ein Nährmedium umfasst.

6. Kapsel nach einem beliebigen der Ansprüche 1 bis 5, **dadurch gekennzeichnet, dass** sie außerdem eine Zwischenhülle umfasst, die vollständig den flüssigen Kern an seinem Umfang einkapselt, wobei die Zwischenhülle selbst vollständig von der gelierten Umhüllung umfänglich eingekapselt ist.

7. Kapsel nach einem beliebigen der Ansprüche 1 bis 6, **dadurch gekennzeichnet, dass** die mindestens eine Zelle mit hematopoetischem Potential eine hematopoetische Stammzelle und/oder eine erythroide Vorläuferzelle und/oder ein erythroider Präkursor ist.

8. Kapsel nach einem beliebigen der Ansprüche 1 bis 7, **dadurch gekennzeichnet, dass** die mindestens eine Zelle mit hematopoetischem Potential oder hematopoetische Stammzelle oder erythroide Vorläuferzelle oder erythroider Vorläufer eine Zelle oder ein humaner Präkursor ist.

9. Verwendung mindestens einer Kapsel nach einem beliebigen der Ansprüche 1 bis 8 für die ex vivo Herstellung von entkernten Erythroidzellen.

10. Verwendung nach Anspruch 9, **dadurch gekennzeichnet, dass** die entkernten Erythroidzellen Retikulozythen und/oder Erythrozyten sind.

11. Herstellungsverfahren ex vivo von entkernten Erythroidzellen, umfassend die Kultur von Zellen mit hematopoetischem Potential, die in mindestens einer Kapsel enthalten sind, wie sie in einem beliebigen der Ansprüche 1 bis 8 definiert ist, bei Bedingungen, die die Herstellung von entkernten Erythroidzellen gestatten.

12. Verfahren nach Anspruch 11, **dadurch gekennzeichnet, dass** die Zellen mit hematopoetischem Potential in einem Kulturmedium kultiviert werden, das umfasst:
e) Insulin mit einer Konzentration zwischen 1 und 50 µg/ml ;
f) Transferrin mit einer Konzentration zwischen 100 und 2000 µg/ml ; und
g) Plasma oder Serum bei einer Konzentration zwischen 1 und 30%.

13. Verfahren nach Anspruch 12, **dadurch gekennzeichnet, dass** das Kulturmedium ebenfalls EPO und/oder SCF und/oder IL-3 und/oder Hydrocortison umfasst.

14. Verfahren nach einem beliebigen der Ansprüche 12 oder 13, **dadurch gekennzeichnet, dass** das Kulturmedium ebenfalls mindestens eine der folgenden Verbindungen umfasst: TPO, FLT3, BMP4, VEGF-A165 und IL-6.

15. Verfahren nach einem beliebigen der Ansprüche 12 bis 14, **dadurch gekennzeichnet, dass** das Kulturmedium ebenfalls ein modifiziertes Iscove Dulbecco Medium umfasst, das durch Glutamin oder ein Glutamin enthaltendes Peptid ergänzt ist.

16. Verfahren nach einem beliebigen der Ansprüche 11 bis 15, **dadurch gekennzeichnet, dass** die entkernten Erythroidzellen Retikulozyten und/oder Erythrozyten sind.

17. Verfahren nach einem beliebigen der Ansprüche 11 bis 16, **dadurch gekennzeichnet, dass** die Zellen mit hematopoetischem Potential hematopoitische Stammzellen und/oder erythroide Vorläuferzellen und/oder erythroide Präkursoren sind.

## Claims

1. A capsule comprising a liquid core, a gelled shell totally encapsulating the liquid core at its periphery, the gelled shell being able to retain the liquid core when the capsule is immersed in a gas, the gelled shell comprising at least one gelled polyelectrolyte and at least one surfactant, **characterized in that** the liquid core comprises at least one cell with hematopoietic potential.

2. The capsule according to claim 1, **characterized in that** it is obtained by applying a method comprising the following steps:
a) separately conveying in a jacket a first physiologically acceptable liquid solution containing at least one cell with hematopoietic potential and of a second liquid solution containing a liquid polyelectrolyte able to be gelled;
b) forming, at the outlet of the jacket, a series of drops, each drop comprising a central core formed with said first solution and a peripheral film formed with said second solution and totally covering the central core;
c) immersing each drop into a gelling solution containing a reagent able to react with the polyelectrolyte of the film so as to have it pass from a liquid state to a gelled state and forming the gelled shell, the central core forming the liquid core;
d) recovering the formed capsules;
the second solution containing at least one surfactant before its contact with the first solution.

3. The capsule according to claim 2, **characterized in that** the ratio of the flow rate of the first solution to the flow rate of the second solution at the outlet of the jacket is comprised between 1 and 200, advantageously between 10 and 200, the gelled shell having a thickness comprised between 0.1% and 10%, advantageously between 0.1% and 2% of the diameter of the capsule, after recovering the formed capsules.

4. The capsule according to any of claims 2 or 3, **characterized in that** the drops formed by co-extrusion in the jacket fall by gravity through a volume of air in the gelling solution.

5. The capsule according to any of claims 2 to 4, **characterized in that** the first physiologically acceptable liquid solution comprises a saline solution, a buffer solution, a physiologically acceptable viscosifying solution, a physiologically acceptable excipient, advantageously a thickener or a rheology modifier, and/or some culture medium.

6. The capsule according to any of claims 1 to 5, **characterized in that** it further comprises an intermediate shell totally encapsulating at its periphery the liquid core, said intermediate shell being itself encapsulated totally at its periphery by the gelled shell.

7. The capsule according to any of claims 1 to 6, **characterized in that** said at least one cell with hematopoietic potential is a hematopoietic stem cell and/or an erythroid progenitor cell and/or an erythroid precursor.

8. The capsule according to any of claims 1 to 7, **characterized in that** said at least one cell with hematopoietic potential or hematopoietic stem cell or erythroid progenitor cell or erythroid precursor is a human cell or precursor.

9. The use of at least one capsule according to any of claims 1 to 8 for *ex vivo* production of enucleated erythroid cells.

10. The use according to claim 9, **characterized in that** the enucleated erythroid cells are reticulocytes and/or erythrocytes.

11. An *ex vivo* method for producing enucleated erythroid cells comprising the culture of cells with hematopoietic potential contained in at least one capsule as defined in any of claims 1 to 8, under conditions allowing the production of enucleated erythroid cells.

12. The method according to claim 11, **characterized in that** the cells with hematopoietic potential are cultivated in a culture medium comprising:
e) insulin at a concentration comprised between 1 and 50 µg/ml ;
f) transferrin at a concentration comprised between 100 and 2,000 µg/ml ; and
g) plasma or serum at a concentration comprised between 1 and 30%.

13. The method according to claim 12, **characterized in that** the culture medium also comprises EPO and/or SCF and/or IL-3 and/or hydrocortisone.

14. The method according to any of claims 12 or 13, **characterized in that** the culture medium also comprises at least one of the following compounds: TPO, FLT3, BMP4, VEGF-A165 and IL-6.

15. The method according to any of claims 12 to 14, **characterized in that** the culture medium also comprises Iscove Dulbecco modified medium, completed with glutamine or a peptide containing glutamine.

16. The method according to any of claims 11 to 15, **characterized in that** the enucleated erythroid cells are reticulocytes and/or erythrocytes.

17. The method according to any of claims 11 to 16, **characterized in that** the cells with hematopoietic potential are hematopoietic stem cells and/or erythroid progenitor cells and/or erythroid precursors.
